(19) Europäisches Patentamt / European Patent Office / Office européen des brevets

(11) **EP 2 351 518 B1**

(12) **FASCICULE DE BREVET EUROPEEN**

(45) Date de publication et mention
de la délivrance du brevet:
**09.05.2018 Bulletin 2018/19**

(51) Int Cl.:
**A61B 5/00** (2006.01)

(21) Numéro de dépôt: **11152692.7**

(22) Date de dépôt: **31.01.2011**

(54) **Sonde optique peropératoire bi-spectrale**

Perioperative bispektrale optische Sonde

Perioperative bi-spectral optical probe

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **02.02.2010 FR 1000401**

(43) Date de publication de la demande:
**03.08.2011 Bulletin 2011/31**

(73) Titulaire: **Commissariat à l'Énergie Atomique
et aux Énergies Alternatives
75015 Paris (FR)**

(72) Inventeurs:
• **Peltie, Philippe
38760, SAINT PAUL DE VARCES (FR)**
• **Berger, Michel
38640, CLAIX (FR)**

(74) Mandataire: **Bréda, Jean-Marc et al
Marks & Clerk France
Conseils en Propriété Industrielle
Immeuble " Visium "
22 Avenue Aristide Briand
94117 Arcueil Cedex (FR)**

(56) Documents cités:
WO-A2-02/07587          US-A- 6 002 137
US-A1- 2005 182 321     US-A1- 2007 232 874

**Description**

Le domaine de l'invention est celui des sondes optiques péropératoires utilisées dans les techniques d'immunophotodétection. Elles servent lors de l'intervention chirurgicale et notamment pour l'ablation de tumeurs.

La technique d'immunophotodétection (acronyme : IPD) a été initiée il y a une dizaine d'années, notamment au Centre de Recherche et de Lutte contre le Cancer de Montpellier. Le principe de cette technique consiste à injecter à un organisme vivant, être humain ou animal, un conjugué anticorps-fluorophore qui se fixe sur les cellules cancéreuses par une réaction anticorps-antigènes. Par exemple, les cancers digestifs sont à même de secréter des antigènes dits carcinoembryonnaires (acronyme : ACE) qui servent de cibles aux anticorps. Au cours de l'opération, le chirurgien doit donc être à même de détecter les fluorophores, indices de la présence de cellules malades. Basiquement, cette détection est assurée par une sonde qui comprend une source laser d'excitation des fluorophores et un photo-détecteur dont la gamme de sensibilité spectrale est adaptée au spectre de fluorescence desdits fluorophores. Cette technique est également connue sous le terme anglo-saxon de « Fluorescence Reflectance Imaging » ou « FRI ».

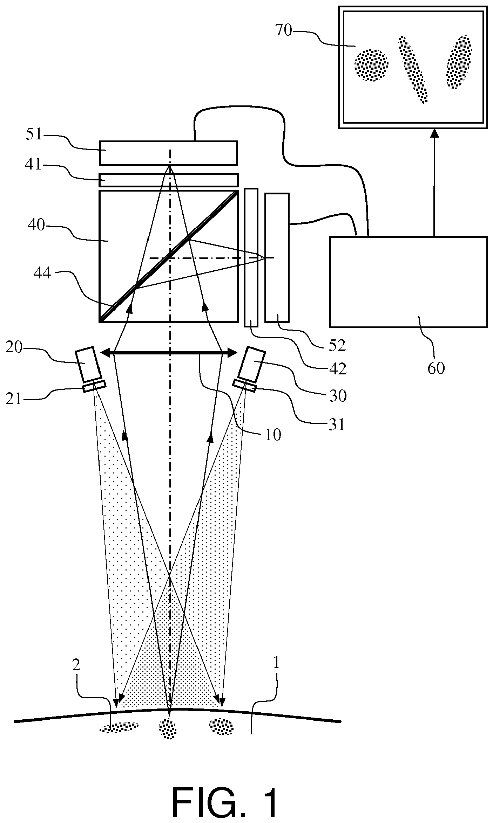

Cette technique présente plusieurs difficultés qu'il faut surmonter pour présenter au chirurgien une image de qualité lui permettant d'avoir le geste le plus efficace possible. Une première difficulté est liée à l'autofluorescence des tissus vivants. On sait que l'autofluorescence des tissus est importante lorsqu'ils sont éclairés par un rayonnement dont le spectre est situé dans le visible. C'est typiquement le cas des salles d'opérations qui sont éclairées soit par la lumière naturelle, soit par la lumière de sources d'éclairage comme les lampes « néon » ou les lampes « halogènes ». Aussi, si l'on ne prend pas de précautions particulières, la lumière environnante est susceptible de parasiter gravement le signal utile de fluorescence, toujours très faible. Un calcul simple permet de comprendre cette difficulté. Dans une sonde peropératoire, la densité de puissance moyenne donnée par la source d'excitation à 700 nm ne dépasse pas 25 $\mu$W/mm$^2$. Si le fluorophore a un rendement quantique de 0.1, une concentration de 10 nM et si, d'autre part, l'épaisseur des tissus traversée n'excède pas 0.1 mm, alors l'intensité de fluorescence vaut approximativement 0.26.10$^{-4}$ de la densité de puissance, soit environ 0.6.10$^{-6}$ mW/mm$^2$. Or, un éclairage de salle d'opération de type scialytique donne une densité de puissance de 0.4 mW/mm$^2$, un million de fois plus puissant. Pour résoudre ce problème, une première solution consiste à utiliser des sources laser d'excitation puissantes de façon à améliorer la fluorescence. Dans ce cas, il faut assurer la sécurité oculaire des personnes présentes pendant l'intervention. Une seconde solution possible est d'utiliser une lumière filtrée bien adaptée aux spécificités de cette technique par « Fluorescence Reflectance Imaging ». Les dispositifs décrits dans la publication de référence US2005/0182321 intitulée «Medical imaging systems» comportent des dispositions de ce type. En effet, le système décrit et représenté en figure 1 de cette publication comporte deux sources d'éclairage filtrées, la première assurant un éclairage visible, la seconde assurant un éclairage d'excitation destiné à la fluorescence des tissus. Ce système comporte également deux caméras ayant un axe optique commun, la première dédiée au rayonnement de fluorescence située dans le proche infrarouge, la seconde dédiée au rayonnement visible.

Un second problème est lié à la surface à examiner qui peut être, par exemple, une cavité abdominale. Généralement, celle-ci est vaste et si l'on utilise une sonde fixe, alors on doit faire une image de l'ensemble de la cavité abdominale. Dans ce cas, la résolution donnée par la caméra est mauvaise et le chirurgien risque de ne pas voir des nodules cancéreux s'ils sont de trop faibles dimensions ou s'ils sont cachés, les nodules de taille importante ayant été détectés soit à l'oeil, soit à la palpation. Aussi, il est préférable d'utiliser une sonde portable que le chirurgien va pouvoir déplacer sur la surface à examiner, l'objectif étant de détecter les nodules dont la taille n'excède pas quelques dixièmes de millimètres. La publication WO 02/061405 intitulée « Method and hand-held device for fluorescence détection» décrit une telle sonde. Cependant, la détection de fluorescence réalisée par cette sonde est rudimentaire. Elle est assurée par un simple photo-détecteur qui ne permet pas de faire une véritable image de la zone à analyser et qui donne simplement une indication de la présence ou non de zones fluorescentes. D'autre part, le problème de l'autofluorescence parasite n'est pas résolu dans une telle sonde.

Un troisième problème est celui de la robustesse de la sonde. Une telle sonde étant destinée à une utilisation intense, il convient que les réglages optiques soient le plus robuste possible.

Un quatrième problème est enfin la qualité de l'image visible des tissus biologiques éclairés par des sources ponctuelles. En effet, il a été constaté que, du fait de l'humidité de ces tissus, ils se comportent comme des surfaces réfléchissantes, ce qui entraîne une réflexion spéculaire significative. Cette réflexion peut considérablement dégrader l'image visible. Le document US2007/232874 décrit le préambule des revendications indépendantes. Le document US4288158 décrit des moyens pour assurer la sécurité oculaire d'un opérateur. La sonde optique selon l'invention permet de pallier ces différents inconvénients. Elle est, en effet, portable, réunit dans un seul module compact à la fois les sources d'excitation et d'éclairage et les deux caméras dédiées d'une part à l'imagerie visible et d'autre part à l'imagerie de fluorescence et comporte enfin des moyens permettant de filtrer efficacement les réflexions spéculaires. Elle comporte également des dispositifs assurant la sécurité oculaire. De plus, il est possible de fixer un écran de visualisation sur cette sonde de façon que le chirurgien puisse visualiser la zone sur laquelle il opère sans avoir à déplacer son regard.

Plus précisément, l'invention a pour premier objet une sonde optique pour applications médicales agencée de façon à pouvoir être tenue d'une seule main, ladite sonde comprenant au moins :

- Une première source d'éclairage dite d'excitation adaptée à provoquer un rayonnement de fluorescence de substances prédéterminées,
- une seconde source d'éclairage visible, la première et la seconde source étant agencées de façon à éclairer une zone commune notée zone d'intervention ;
- un premier capteur matriciel photosensible ;
- un second capteur matriciel photosensible ;

les premier et second capteurs matriciels photosensibles étant agencés de façon que, lorsque la sonde est disposée à une distance prédéterminée de la zone d'intervention, l'image dans le spectre de fluorescence de ladite zone se forme sur la surface photosensible du premier capteur matriciel et l'image dans le spectre visible de ladite zone se forme sur la surface photosensible du second capteur matriciel, caractérisée en ce que la seconde source d'éclairage visible comporte un filtre polarisant, un analyseur étant alors disposé en amont du second capteur matriciel photosensible, la direction de polarisation de l'analyseur étant alors perpendiculaire à la direction de polarisation du filtre polarisant

Avantageusement, ladite sonde comprend :

- un seul objectif optique commun ;
- un ensemble prismatique séparateur monobloc et des filtres spectraux ;

l'objectif optique, le prisme séparateur monobloc, les filtres spectraux, les premier et second capteurs matriciels photosensibles étant agencés de façon que, lorsque l'objectif optique est disposé à une distance prédéterminée de la zone d'intervention, l'image dans le spectre de fluorescence de ladite zone donnée par l'objectif se forme sur la surface photosensible du premier capteur matriciel et l'image dans le spectre visible de ladite zone donnée par l'objectif se forme sur la surface photosensible du second capteur matriciel, l'analyseur étant alors disposé entre le prisme séparateur monobloc et le second capteur matriciel photosensible.

Avantageusement, le premier capteur matriciel est associé à un premier filtre, transmettant uniquement dans la bande de fluorescence, et que le second capteur matriciel est associé à un second filtre, transmettant des longueurs d'ondes visibles à l'exception de celles comprises dans la bande de fluorescence.

L'invention concerne également une sonde optique pour applications médicales agencée de façon à pouvoir être tenue d'une seule main, ladite sonde comprenant au moins :

- Une première source d'éclairage dite d'excitation adaptée à provoquer un rayonnement de fluorescence de substances prédéterminées,
- une seconde source d'éclairage visible, la première et la seconde source étant agencées de façon à éclairer une zone commune notée zone d'intervention ;
- un objectif optique ;
- un ensemble prismatique séparateur monobloc et des filtres spectraux ;
- un capteur matriciel photosensible ;

l'objectif optique, le prisme séparateur monobloc, les filtres spectraux, le capteur matriciel photosensible étant agencés de façon que, lorsque l'objectif optique est disposé à une distance prédéterminée de la zone d'intervention, l'image dans le spectre de fluorescence de ladite zone donnée par l'objectif se forme sur la première partie de la surface photosensible du capteur matriciel et l'image dans le spectre visible de ladite zone donnée par l'objectif se forme sur une seconde partie de la surface photosensible du capteur matriciel, caractérisée en ce que la seconde source d'éclairage visible comporte un filtre polarisant, un analyseur étant alors disposé en amont de la seconde partie du capteur matriciel photosensible, la direction de polarisation de l'analyseur étant alors perpendiculaire à la direction de polarisation du filtre polarisant

Avantageusement, l'ensemble prismatique séparateur comporte un cube séparateur et un prisme de renvoi et une lame de compensation, le cube séparateur comportant un traitement dichroïque réfléchissant le rayonnement visible et transmettant le rayonnement situé dans la bande de fluorescence ou réciproquement.

Avantageusement, l'ensemble prismatique séparateur est un prisme de «Koster» composé de deux prismes identiques en équerre, la face commune aux deux prismes comprenant un traitement dichroïque réfléchissant le rayonnement visible et transmettant le rayonnement situé dans la bande de fluorescence ou réciproquement. Selon l'invention, la première source d'éclairage dite d'excitation est une source laser dont l'émission spectrale correspond au spectre d'excitation du fluorophore. Selon l'invention, la sonde comporte des moyens de mesure de l'inclinaison de la sonde optique et des moyens de coupure de la source laser lorsque l'inclinaison de la sonde optique dépasse une valeur prédéterminée.

Avantageusement, la seconde source d'éclairage est au moins une diode électroluminescente dite « blanche » comprenant un filtre coupant le spectre de fluorescence.

Avantageusement, la seconde source d'éclairage comporte une pluralité de diodes blanches filtrées disposées de façon régulière autour de l'objectif optique.

Enfin, la sonde peut comporter un imageur agencé de façon à afficher soit l'image dans le spectre visible de la zone d'intervention, soit l'image dans le spectre de fluorescence de la zone d'intervention, soit une superposition de ces deux images, lesdites images étant issues du ou des capteurs matriciels photosensibles.

L'invention sera mieux comprise et d'autres avantages apparaîtront à la lecture de la description qui va suivre donnée à titre non limitatif et grâce aux figures annexées parmi lesquelles :

La figure 1 représente un premier mode de réalisation d'une sonde selon l'invention et des dispositifs annexes ;

La figure 2 représente un second mode de réalisation d'une sonde selon l'invention, seule la partie supérieure de la sonde est représentée sur cette figure ;

La figure 3 représente une variante de ce second mode de réalisation d'une sonde selon l'invention ;

La figure 4 représente le principe général du dispositif permettant d'assurer la sécurité oculaire de la sonde ;

La figure 5 représente une sonde selon l'invention comportant un afficheur ;

La figure 6 représente un mode de réalisation de l'éclairage visible ;

La figure 7 représente la répartition spectrale des différents filtrages optiques nécessaires dans une sonde selon l'invention.

Comme il a été dit, les sondes selon l'invention comportent deux voies, une première voie dédiée au rayonnement de fluorescence et une seconde voie dédiée au rayonnement visible polarisé. Il existe différentes architectures optiques possibles permettant de réaliser ces deux voies.Une première architecture possible consiste à réaliser deux voies distinctes comportant chacune leur propre source, leur système optique et leur capteur matriciel, chaque voie formant une image de la même zone d'intervention. La voie visible fonctionne alors en lumière polarisée.

Une seconde architecture optique possible permettant d'assurer une plus grande compacité de la sonde est de réaliser une combinaison optique comportant un objectif optique unique commun aux deux voies.

La figure 1 représente le schéma d'un premier mode de réalisation d'une sonde optique peropératoire selon cette architecture. Sur cette figure et sur les suivantes, les dimensions ne sont pas nécessairement représentatives de celles d'une sonde réelle, l'objectif étant de montrer les principes généraux d'implantation opto-mécaniques des différents éléments et le parcours des rayons lumineux à travers les éléments optiques. Ne sont pas non plus représentés sur cette figure et les suivantes le carter mécanique entourant la sonde et tenu par l'utilisateur ainsi que les différents supports mécaniques permettant de maintenir et de positionner les différents composants optiques. La mise en place de ses différents éléments ne pose pas de difficultés particulières pour l'homme du métier.

Sur la figure 1, les éléments polarisants ne sont pas représentés dans la mesure où ils peuvent se situer à différents endroits de la sonde ou être intégrés à d'autres filtres optiques. On se reportera sur ce point particulier au chapitre de la description intitulé « Caractéristiques de la source visible ou source blanche ».

La sonde est représentée en utilisation opérationnelle, c'est-à-dire tenue par un opérateur au-dessus d'une zone d'intervention.

La sonde selon l'invention comprend essentiellement :

- Une première source d'éclairage dite d'excitation 20, le rayonnement spectral de cette première source est filtré au moyen du filtre optique 21 ;
- une seconde source d'éclairage visible ou « blanche » 30 filtrée au moyen du filtre optique 31, la première et la seconde source étant agencées de façon à éclairer une zone commune notée zone d'intervention 1. Sur la figure 1, les différents rayonnements émis sont représentés en pointillés ;
- un objectif optique 10 représenté classiquement par une double flèche ;
- un ensemble prismatique séparateur monobloc 40 et des filtres spectraux 41 et 42 ;
- un premier capteur matriciel photosensible 51 ;
- un second capteur matriciel photosensible 52 ;

La première et la seconde source constituent la voie d'émission de la sonde. L'objectif, les filtres spectraux et les capteurs matriciels composent les deux voies de réception de la sonde optique.

La zone d'intervention 1 peut être, par exemple, une partie d'une cavité abdominale susceptible de comporter des tissus cancéreux 2. Elle a été préalablement traitée au moyen d'une injection d'un conjugué anticorps-fluorophore qui s'est fixé sur les cellules malades 2. La source d'excitation 20 émet un rayonnement spectral dans une première bande spectrale dite d'excitation qui éclaire la zone d'intervention 1, permettant d'obtenir la fluorescence des cellules malades marquées par le fluorophore. Le rayonnement de fluorescence est émis dans une seconde bande spectrale dite de fluorescence. Généralement, l'essentiel du spectre de fluorescence est émis dans le rouge et le proche-infrarouge.

La zone d'intervention est également éclairée par de la lumière visible dite « blanche » venant de la source 30. Cette lumière est filtrée et ne comporte plus de rayonnement situé dans le rouge ou le proche infrarouge. Ainsi, il est possible de séparer spectralement le spectre visible du spectre de fluorescence. Aussi, la sonde comporte deux voix de réception, la première dédiée au rayonnement visible, la seconde au rayonnement de fluorescence. Ces deux voies comportent un objectif optique commun 10 qui forme d'une part, l'image de fluorescence de la zone d'intervention 1 sur le premier capteur matriciel photosensible 51 et l'image visible de la zone d'intervention sur le second capteur matriciel photosensible 52, la séparation spectrale des deux images est assurée par un traitement dichroïque 44 disposé à l'intérieur du prisme séparateur monobloc 40. On peut inverser les voies de réception pour obtenir une meilleure implantation. Des filtres spectraux 41 et 42 supplémentaires permettent de parfaitement séparer les spectres. Les images électroniques fournies par ces deux capteurs sont traitées par une unité de traitement électronique 60, indépendante de la sonde qui peut assurer les traitements d'images usuels et qui renvoie les images traitées vers un écran de visualisation 70 qui peut afficher soit l'une des deux images visible ou de fluorescence, soit une fusion de ces deux images. La fusion de ces deux images peut être l'image dans le spectre visible sur laquelle on superpose les parties les plus intenses de l'image de fluorescence, ces parties pouvant être par exemple colorisées.

On peut, bien entendu, utiliser comme ensemble prismatique une simple lame semi-réfléchissante dichroïque pour séparer spectralement les deux images. Cependant, il est préférable d'utiliser un cube séparateur 40 comme on le voit sur la figure 1 dont la face interne comporte un traitement dichroïque 44. En effet, le cube séparateur présente de nombreux avantages. D'une part, il se comporte comme une lame de verre épaisse et permet de réduire l'encombrement des faisceaux optiques, la réfraction à l'intérieur du cube entraînant une moindre divergence des faisceaux. Ensuite, il permet d'augmenter le tirage de l'objectif, permettant d'utiliser des optiques standards. On démontre que si e est l'épaisseur du cube et n son indice de réfraction, l'augmentation

de tirage $\delta$T vaut $e.\left(1 - \dfrac{1}{n}\right)$. Enfin, il est possible de coller les filtres et les capteurs sur les faces planes du cube. D'une part, on supprime de possibles images parasites et d'autre part, on réalise ainsi un composant qui sera très peu sensible aux chocs mécaniques ou aux variations thermiques, ce qui est essentiel pour une sonde qui est constamment manipulée. Le collage des filtres et des capteurs, voire de l'objectif, sur le cube permettent d'accroître la robustesse de la sonde, et de prévenir d'éventuels déréglages intempestifs.

Dans la version de base illustrée en figure 1, les capteurs 51 et 52 sont différents. On peut ainsi spécifiquement adapter leur réponse spectrale au rayonnement reçu. Dans une variante, il est possible d'utiliser un capteur unique 53 pour réaliser les images visible et de fluorescence. L'image dans le spectre de fluorescence de ladite zone se forme sur la première partie 54, par exemple une première moitié de la surface photosensible du capteur matriciel 53, et l'image dans le spectre visible de ladite zone d'intervention se forme sur une seconde partie 55, par exemple une seconde moitié, de la surface photosensible du capteur matriciel. Dans ce cas, le prisme séparateur doit avoir une disposition particulière de façon à assurer la focalisation des deux images de la zone d'intervention dans le plan de la surface photosensible du capteur matriciel.

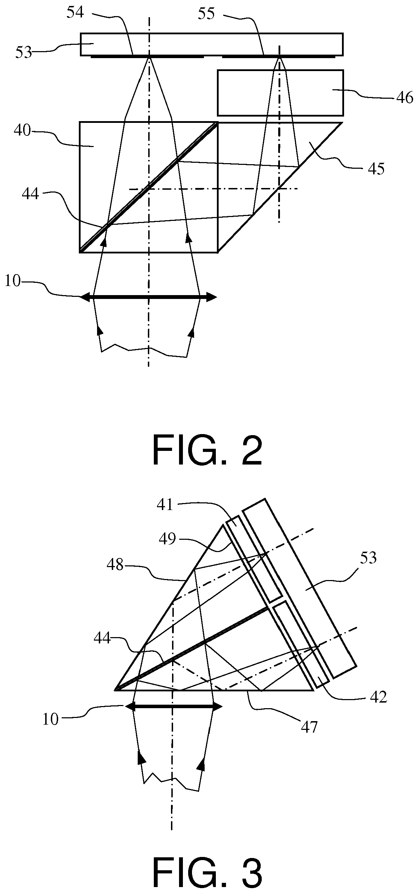

Dans une premier mode de réalisation représenté en figure 2, le prisme séparateur comporte un cube séparateur 40, un prisme de renvoi 45 et une lame de compensation 46, cette lame pouvant également faire office de filtre de la lumière fluorescente, ou être collée à un tel filtre. Le cube séparateur 40 comporte comme précédemment une face interne dichroïque 44. Le prisme de renvoi 45 permet d'orienter l'axe optique sur la voie en réflexion de la face dichroïque selon un axe parallèle à celui de la voie en transmission. Il fonctionne naturellement en réflexion totale. La lame compensatrice 46 permet d'égaliser les tirages optiques sur les deux voies de réception. En effet, il est nécessaire de compenser le trajet optique perdu dans le prisme de renvoi 45 par une augmentation du tirage dû à la lame compensatrice 46. Bien que non représentés sur la figure 2, ce mode de réalisation peut comprendre un filtre 41, adapté à la bande spectrale de fluorescence, ainsi qu'un filtre 42, dont la bande spectrale comprend des longueurs d'onde visibles à l'exception de celles contenues dans la bande de fluorescence.

Dans un second mode de réalisation représenté en figure 3, le prisme séparateur est un prisme de « Koster » composé de deux prismes identiques, la face commune aux deux prismes 44 comprenant un traitement dichroïque réfléchissant le rayonnement visible et transmettant le rayonnement situé dans le proche infrarouge. Chaque prisme a une section en forme de triangle rectangle. La propagation des rayons lumineux est la suivante : les rayons lumineux issus de l'objectif 10 pénètrent dans le prisme de « Koster » par la face inférieure 47, sont séparés par le traitement dichroïque 44, sont réfléchis par réflexion totale sur les faces 47 et 48 et sortent en incidence quasi-normale par la face 49 où ils peuvent être filtrés par les filtres 41 et 42 avant de se focaliser sur le capteur photosensible unique 53.

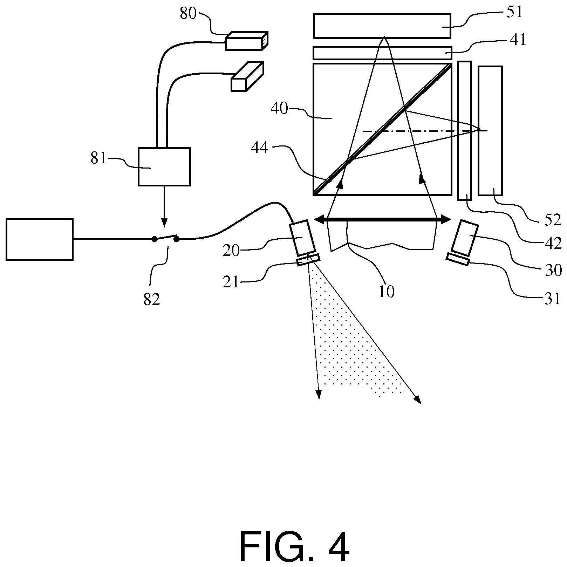

Comme il a été dit, les sources d'excitation sont généralement des sources laser suffisamment puissantes pour provoquer une fluorescence décelable. Aussi, il est important d'assurer la sécurité oculaire de l'opérateur ou du personnel pratiquant l'intervention. Une solution simple est exposée en figure 4. On fixe sur la sonde deux inclinomètres 80 ou des dispositifs équivalents orientés à 90 degrés l'un de l'autre. Ces inclinomètres sont reliés à un dispositif de traitement 81 qui compare les mesures d'inclinaison de la sonde à des valeurs d'inclinaison prédéterminées dites de sécurité. Lorsque les mesures atteignent ou dépassent lesdites valeurs, le dispositif de traitement 81 coupe le faisceau laser. La valeur de sécurité peut correspondre, par exemple, à une mise à l'horizontale de la sonde. Cette coupure est symboliquement représentée par l'interrupteur 82. On peut soit couper électriquement l'alimentation du laser, soit introduire un cache devant le faisceau si l'on ne souhaite pas interrompre brutalement l'émission laser.

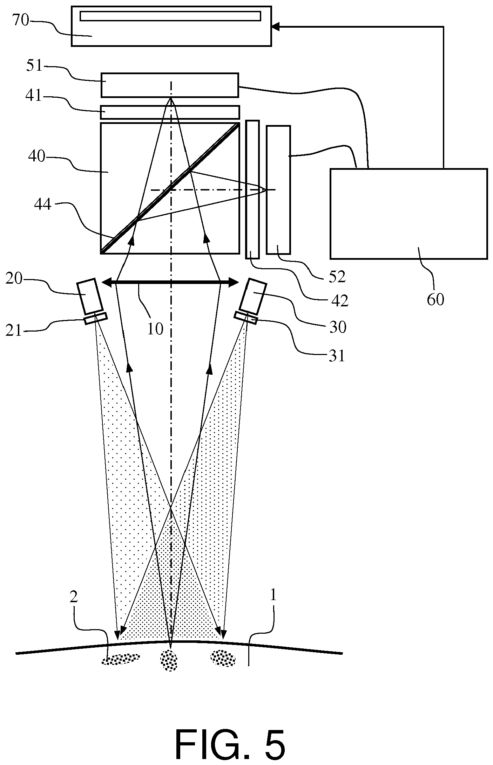

Les images fournies par le ou les capteurs sont affichées sur un écran de visualisation 70. Cet écran peut être un écran fixé à demeure dans la salle d'opération. On peut également fixer un écran de petite taille directement sur la sonde optique de façon que l'opérateur ait constamment sous les yeux une image de la zone d'intervention, c'est ce qui est illustré en figure 5. Cet écran 70 peut être, par exemple, un écran plat à cristaux liquides. Cet écran peut également être filtré de façon à ne pas perturber la fluorescence.

A titre d'exemple non limitatif, les caractéristiques optiques, photométriques, géométriques des différents composants optiques et opt-électroniques peuvent être les suivants.

### Caractéristiques de la source d'excitation

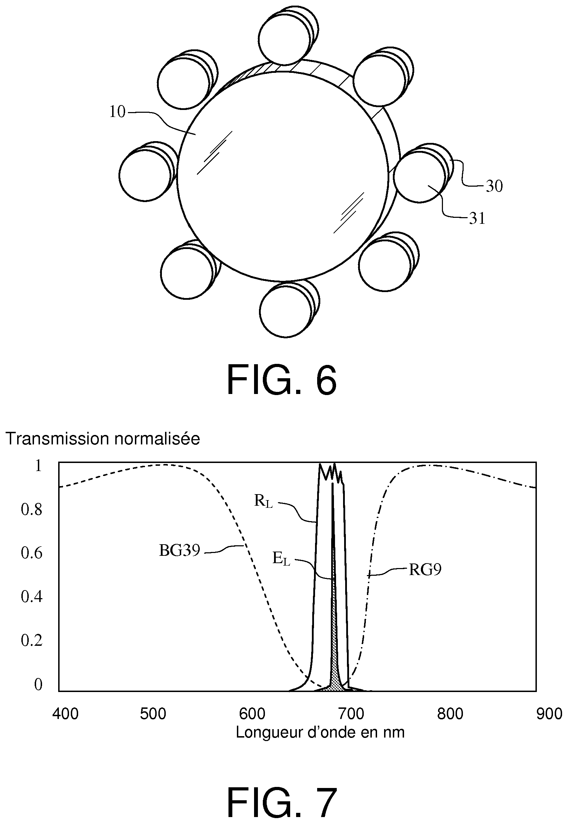

La source d'excitation 20 est adaptée au spectre d'excitation du fluorophore utilisé. Par spectre d'excitation, on entend bien sur le spectre d'excitation de fluorescence du fluorophore. Dans le cas où le colorant est un dérivé du vert d'indocyanine, connu sous l'acronyme ICG ayant une absorption optimale à 686 nm et une émission à 704 nm, la source peut être un laser trié pour émettre à 685 nm ± 5 nm. Ce laser est régulé en température par effet Peltier de façon à stabiliser la longueur d'onde d'émission à la longueur d'onde souhaitée. A titre d'exemple, une variation de la température d'un degré permet de déplacer le pic d'émission de 0.2 nm. La figure 7 représente un certain nombre de répartitions spectrales en fonction de la longueur d'onde, celle-ci étant comprise entre 400 et 900 nm. La courbe en gris $E_L$ centrée sur 685 nm représente l'émission spectrale du laser. Pour améliorer la pureté spectrale de l'émission laser, on peut utiliser un filtre interférentiel. Cela permet d'éviter la présence d'émissions secondaires de la source d'excitation dans la bande spectrale de fluorescence. La courbe de transmission spectrale $R_L$ de ce filtre est représentée sur la figure 7. Elle est centrée sur 685 nm et a une largeur à mi-hauteur de 30 nm. Par nature, la courbe de filtrage de ce type de filtre interférentiel est très sensible à l'incidence des rayons lumineux qui traversent le filtre. Aussi, il est préférable de les utiliser en lumière collimatée. Il suffit de placer une lentille collimatrice, par exemple à gradient d'indice devant la source d'émission et de disposer le filtre interférentiel derrière cette lentille. On peut ensuite disposer soit une autre lentille, soit un diffuseur pour obtenir la divergence souhaitée permettant d'éclairer de façon homogène la zone d'intervention.

De façon à éviter d'augmenter le poids et le volume de la sonde qui doit facilement être tenue par l'utilisateur, il est préférable de déporter le laser hors de la sonde et d'amener le rayonnement d'excitation au moyen d'une fibre optique. Pour obtenir une fluorescence facilement détectable, la puissance d'excitation doit être comprise entre 0.25 W et 0.5 W. Dans ce cas, il est recommandé d'utiliser un dispositif de sécurité oculaire à clinomètres tel qu'il a été précédemment décrit.

### Caractéristiques de la source visible ou source blanche.

Il est préférable d'utiliser des diodes électroluminescentes blanches filtrées pour réaliser cet éclairage. Les diodes blanches permettent d'obtenir des puissances lumineuses élevées dans un encombrement réduit. De façon à obtenir une répartition d'éclairage très homogène, on peut utiliser plusieurs sources blanches 30 réparties uniformément autour de l'optique d'entrée de l'objectif 10 comme représenté en figure 6 où, à titre d'exemple, 8 diodes électroluminescentes filtrées entourent l'objectif optique. Il est très souhaitable que ces diodes soient filtrées de façon à éliminer la bande spectrale de leur spectre d'émission correspondant au spectre d'émission du fluorophore, ou spectre de fluorescence (rouge et infra rouge dans le cas présent) qui viendrait perturber le rayonnement de fluorescence généralement très faible. On peut, à cet effet utiliser des filtres 31 de type « BG39 » de la société SCHOTT qui fonctionnent par absorption et sont donc peu sensibles à l'inclinaison des rayons lumineux et qui présentent l'avantage de conserver un bon rendu des couleurs. Lorsque le dispositif comporte une couronne de diodes, il peut être intéressant de disposer un filtre unique en anneau ou en portion d'anneau devant l'ensemble des diodes d'éclairage. La courbe de transmission spectrale BG39 de ce filtre est représentée sur la figure 7.

Compte-tenu de la faible intensité du rayonnement de fluorescence, il est préférable d'utiliser uniquement la source d'éclairage filtrée pour éclairer la zone d'intervention et de couper totalement l'éclairage scialytique de la pièce où se déroule l'intervention, celle-ci étant de toute façon faiblement éclairée par l'écran d'affichage qui peut également être filtré s'il est de dimensions réduites.

Les tissus biologiques sont des milieux humides et peuvent, de surcroît être humidifiés en cours d'intervention par du sérum. Des essais expérimentaux ont montré que lorsqu'ils sont éclairés par des sources de lumière blanche telles que décrites ci-dessus, ils peuvent se comporter comme une surface réfléchissante, et entraîner une réflexion spéculaire importante, susceptible d'apparaître sur l'image acquise dans le spectre visible, occasionnant alors une gêne pour l'utilisateur. En effet, les taches de réflexion spéculaire apparaissant sur l'image visible sont floues, car l'objet virtuel auquel elles correspondent est approximativement à une distance double de la distance de mise au point. Il a été constaté que ces taches peuvent être très intenses par rapport à la lumière blanche ayant diffusé, produisant des saturations locales sur l'imageur. On remédie à ce problème en incorporant un filtre polarisant à proximité de la source de lumière blanche, et en plaçant un analyseur devant la matrice photosensible correspondant à l'image visible, la direction de polarisation de cet analyseur étant perpendiculaire à celle du filtre polarisant placé à proximité de la source d'éclairage blanc. Ce perfectionnement permet d'éliminer, en amont de l'imageur, le signal dû aux réflexions spéculaires car la lumière réfléchie spéculaire conserve sa polarisation, à l'inverse de la lumière réfléchie diffuse, cette dernière étant dépolarisée.

On peut par exemple utiliser un premier filtre de polarisation linéaire de marque « Vikuiti » et de type HN 32, et un analyseur constitué par un filtre de conception identique, orienté perpendiculairement au premier filtre. Afin de ne pas influer sur le signal de fluorescence, l'analyseur est disposé après la séparation des signaux visible et de fluorescence. De préférence, l'analyseur est disposé contre l'imageur visible 52 ou contre le filtre 42 associé à cet imageur. On choisit de préférence un filtre polarisant et un analyseur de faible épaisseur, typiquement de quelques centaines de $\mu$m.

**Caractéristiques opto-mécaniques et photométriques**

La zone d'intervention a un diamètre d'environ 80 mm. La distance de travail d'une sonde optique tenue à la main, c'est-à-dire la distance qui sépare l'objectif de la zone d'intervention est de l'ordre de 120 à 150 mm. Pour garder une compacité importante, on choisit des capteurs matriciels photosensibles dont la diagonale est proche de 8 mm. De ces dimensions, on en déduit la focale de l'objectif 10 qui doit faire environ 12.5 mm. L'objectif retenu peut être dérivé des objectifs standard utilisés pour la prise de vue photographique. L'objectif doit être conçu de façon que l'on puisse interposer l'ensemble prismatique séparateur entre le dernier dioptre de l'objectif et la ou les surfaces des capteurs photosensibles. Cette contrainte ne pose pas de problèmes particuliers dans la mesure où l'ensemble séparateur assimilable à une lame de verre épaisse introduit naturellement une augmentation du tirage de l'objectif.

Comme il a été dit, le filtrage spectral des deux voies de réception doit être particulièrement soigné de façon à parfaitement séparer le spectre de fluorescence du spectre visible. Cette séparation n'est pas nécessairement parfaitement assurée par le traitement dichroïque du prisme séparateur. Aussi, il peut être judicieux de disposer devant chaque capteur matriciel photosensible un filtre optique transmettant uniquement soit le visible, mais à l'exception de la bande spectrale de l'émission fluorescente (ou bande de fluorescence), correspondant au spectre de fluorescence, soit uniquement dans la bande spectrale de l'émission fluorescence. Dans de nombreuses applications, cette bande de fluorescence se situe dans le rouge ou dans le proche infra-rouge, mais l'invention peut être aisément adaptée à d'autres spectres de fluorescence. Dans le premier cas, on peut utiliser un filtre de type « BG39 », dans le second cas, on peut utiliser un filtre RG9, également de la société SCHOTT. On se reportera aux fiches techniques de cette société pour obtenir toutes les caractéristiques optiques de ces filtres.

A titre d'information, la courbe de transmission spectrale RG9 de ce filtre est représentée sur la figure 7. De façon à améliorer la compacité, la qualité et la robustesse de l'ensemble optique, il est avantageux de coller les filtres de sélection spectrale sur les faces de l'ensemble prismatique séparateur en regard des surfaces photosensibles des capteurs.

**Caractéristiques des capteurs matriciels**

Les capteurs peuvent être de type CCD, acronyme de « Charge Coupled Device » ou CMOS, acronyme de « Complementary Metal Oxide Semiconductor ».

Lorsque la sonde comporte deux capteurs différents, le capteur matriciel disposé sur la voie de fluorescence peut être monochrome. Il doit avoir une bonne résolution et une bonne sensibilité dans le spectre situé dans le rouge et le proche infrarouge. A titre d'exemple, le capteur matriciel de référence « ICX285AL » de la société SONY répond à ce besoin. Il possède une surface utile de longueur 8.3 mm et de largeur 6.6 mm comportant 1292 lignes de 1024 pixels, chaque pixel carré mesurant 6.45 $\mu$m de côté. Il offre un bon rendement quantique à 700 nm. Ce capteur ne nécessite pas de refroidissement et peut fonctionner à des cadences de plusieurs images par seconde. Pour toutes informations complémentaires, il est possible de se reporter à la fiche du fabricant.

Le capteur matriciel disposé sur la voie visible est nécessairement un capteur couleur et ses caractéristiques dimensionnelles doivent être voisines de celles du capteur matriciel disposé sur la voie de fluorescence. A titre d'exemple, un capteur matriciel de type CMOS de la société canadienne PIXELLINK répond à ce besoin. Il possède une surface utile de longueur 8.6 mm et de largeur 6.8 mm comportant 1280 lignes de 1024 pixels, chaque pixel carré mesurant 6.7 $\mu$m de côté. Il fonctionne à une cadence de 24 images par seconde.

Lorsque la sonde comporte un seul capteur, celui-ci doit nécessairement être un capteur couleur dont la surface photosensible est de forme rectangulaire. A titre d'exemple, le capteur matriciel de référence « ICX412AQ » de la société SONY répond à ce besoin. Pour toutes informations complémentaires, il est possible de se reporter à la fiche du fabricant.

De façon à améliorer la compacité, la qualité et la robustesse de l'ensemble optique, il est également avantageux de coller les capteurs sur les filtres de sélection spectrale. Il est bien entendu, très important que les images visibles et de fluorescence se superposent parfaitement. L'alignement et le réglage de superposition des deux surfaces photosensibles peuvent se faire au moyen d'un stéréomicroscope avec une grande précision et ne présentent pas de difficultés particulières. Là encore, il est préférable de coller les capteurs sur les éléments optiques pour conserver les positions de superposition. Il est également préférable de déporter toutt ou partie de l'électronique de commande des capteurs de façon à alléger la sonde et à réduire son encombrement.

Au final, il est possible de réaliser une sonde optique portable dont la longueur ne dépasse pas 170 mm et dont la section rectangulaire a comme dimensions 56x43 mm et dont le poids ne dépasse pas 500 g.

## Revendications

1. Sonde optique pour applications médicales agencée de façon à pouvoir être tenue d'une seule main, ladite sonde comprenant au moins :

   - Une première source d'éclairage dite d'excitation (20) adaptée à provoquer un rayonnement de fluorescence de substances prédéterminées,
   - une seconde source d'éclairage visible (21), la première et la seconde source étant agencées de façon à éclairer une zone commune (1) notée zone d'intervention ;
   - un premier capteur matriciel photosensible (51) ;
   - un second capteur matriciel photosensible (52) ;

   les premier et second capteurs matriciels photosensibles étant agencés de façon que, lorsque la sonde est disposée à une distance prédéterminée de la zone d'intervention, l'image dans le spectre de fluorescence de ladite zone se forme sur la surface photosensible du premier capteur matriciel et l'image dans le spectre visible de ladite zone se forme sur la surface photosensible du second capteur matriciel, la seconde source d'éclairage visible comporte un filtre polarisant, un analyseur étant alors disposé en amont du second capteur matriciel photosensible, la direction de polarisation de l'analyseur étant alors perpendiculaire à la direction de polarisation du filtre polarisant,
   **caractérisé en ce que** la première source d'éclairage dite d'excitation (20) est une source laser dont l'émission spectrale correspond au spectre d'excitation et **en ce que** la sonde comporte des moyens de mesure de l'inclinaison (80, 81) de la sonde optique et des moyens de coupure (82) de la source laser (20) lorsque l'inclinaison de la sonde optique dépasse une valeur prédéterminée.

2. Sonde optique selon la revendication 1, ladite sonde comprenant :

   - un seul objectif optique (10) commun ;
   - un ensemble prismatique séparateur monobloc (40) et des filtres spectraux (41, 42) ;

   l'objectif optique, le prisme séparateur monobloc, les filtres spectraux, les premier et second capteurs matriciels photosensibles étant agencés de façon que, lorsque l'objectif optique est disposé à une distance prédéterminée de la zone d'intervention, l'image dans le spectre de fluorescence de ladite zone donnée par l'objectif se forme sur la surface photosensible du premier capteur matriciel et l'image dans le spectre visible de ladite zone donnée par l'objectif se forme sur la surface photosensible du second capteur matriciel, l'analyseur étant alors disposé entre le prisme séparateur monobloc et le second capteur matriciel photosensible.

3. Sonde optique selon la revendication 2, telles que le premier capteur matriciel (51) est associé à un premier filtre (41), transmettant uniquement dans la bande de fluorescence, et que le second capteur matriciel (52) est associé à un second filtre (42), transmettant des longueurs d'ondes visibles à l'exception de celles comprises dans la bande de fluorescence.

4. Sonde optique pour applications médicales agencée de façon à pouvoir être tenue d'une seule main, ladite sonde comprenant au moins :

   - Une première source d'éclairage dite d'excitation (20) adaptée à provoquer un rayonnement de fluorescence de substances prédéterminées,
   - une seconde source d'éclairage visible (21), la première et la seconde source étant agencées de façon à éclairer une zone commune (1) notée zone d'intervention ;
   - un objectif optique (10) ;
   - un ensemble prismatique séparateur monobloc (40) et des filtres spectraux (41, 42) ;
   - un capteur matriciel photosensible (53) ;

   l'objectif optique, le prisme séparateur monobloc, les filtres spectraux, le capteur matriciel photosensible étant agencés de façon que, lorsque l'objectif optique est disposé à une distance prédéterminée de la zone d'intervention, l'image dans le spectre de fluorescence de ladite zone donnée par l'objectif se forme sur la première partie (54) de la surface photosensible du capteur matriciel et l'image dans le spectre visible de ladite zone donnée par l'objectif se forme sur une seconde partie (55) de la surface photosensible du capteur matriciel,
   la seconde source d'éclairage visible comporte un filtre polarisant, un analyseur étant alors disposé en amont de la seconde partie du capteur matriciel photosensible, la direction de polarisation de l'analyseur étant alors perpendiculaire à la direction de polarisation du filtre polarisant,
   **caractérisé en ce que** la première source d'éclairage dite d'excitation (20) est une source laser dont l'émission spectrale correspond au spectre d'excitation et **en ce que** la sonde comporte des moyens de mesure de l'inclinaison (80, 81) de la sonde optique et des moyens de coupure (82) de la source laser (20) lorsque l'inclinaison de la sonde optique dépasse une valeur prédéterminée.

5. Sonde optique selon la revendication 4, **caractérisé en ce que** l'ensemble prismatique séparateur comporte un cube séparateur (40) et un prisme de renvoi (45) et une lame de compensation (46), le cube séparateur comportant un traitement dichroïque (44) réfléchissant le rayonnement visible et transmettant le rayonnement situé dans la bande de fluorescence ou réciproquement.

6. Sonde optique selon la revendication 5, **caractérisé en ce que** l'ensemble prismatique séparateur est un prisme de « Koster » composé de deux prismes identiques en équerre, la face commune aux deux prismes comprenant un traitement dichroïque (44) réfléchissant le rayonnement visible et transmettant le rayonnement situé dans la bande de fluorescence ou réciproquement.

7. Sonde optique selon l'une des revendications précédentes, **caractérisé en ce que** la seconde source d'éclairage (30) est au moins une diode électroluminescente dite « blanche » comprenant un filtre coupant le spectre de fluorescence.

8. Sonde optique selon la revendication 7, **caractérisé en ce que** la seconde source d'éclairage comporte une pluralité de diodes blanches filtrées disposées de façon régulière autour d'un objectif optique.

9. Sonde optique selon l'une des revendication précédentes, **caractérisé en ce que** la sonde comporte un imageur (70) agencé de façon à afficher soit l'image dans le spectre visible de la zone d'intervention, soit l'image dans le spectre de fluorescence de la zone d'intervention, soit une superposition de ces deux images, lesdites images étant issues du ou des capteurs matriciels photosensibles.

**Patentansprüche**

1. Optische Sonde für medizinische Anwendungen, so ausgestattet, dass sie in einer einzigen Hand gehalten werden kann, wobei die Sonde wenigstens Folgendes umfasst:

   - eine erste Beleuchtungsquelle, genannt Anregungsbeleuchtungsquelle (20), ausgelegt zum Hervorrufen einer Fluoreszenzstrahlung von vorbestimmten Substanzen,
   - eine zweite sichtbare Beleuchtungsquelle (21), wobei die erste und die zweite Quelle zum Beleuchten einer gemeinsamen Zone (1), Eingriffszone genannt, ausgelegt sind;
   - einen ersten fotoempfindlichen Matrixsensor (51);
   - einen zweiten fotoempfindlichen Matrixsensor (52);

   wobei der erste und der zweite fotoempfindliche Matrixsensor so ausgestattet sind, dass, wenn die Sonde in einem vorbestimmten Abstand von der Eingriffszone angeordnet wird, das Bild im Fluoreszenzspektrum der Zone auf der fotoempfindlichen Fläche des ersten Matrixsensors entsteht und das Bild im sichtbaren Spektrum der Zone auf der fotoempfindlichen Fläche des zweiten Matrixsensors entsteht, wobei die zweite sichtbare Beleuchtungsquelle ein Polarisationsfilter umfasst, wobei ein Analysator dann oberhalb des zweiten fotoempfindlichen Matrixsensors angeordnet wird, wobei die Polarisationsrichtung des Analysators dann lotrecht zur Polarisationsrichtung des Polarisationsfilters ist, **dadurch gekennzeichnet, dass** die erste Beleuchtungsquelle, genannt Anregungsbeleuchtungsquelle (20), eine Laserquelle ist, deren Spektralemission dem Anregungsspektrum entspricht, und dadurch, dass die Sonde Mittel (80, 81) zum Messen der Neigung der optischen Sonde und Mittel (82) zum Beschneiden der Laserquelle (20) umfasst, wenn die Neigung der optischen Sonde einen vorbestimmten Wert überschreitet.

2. Optische Sonde nach Anspruch 1, wobei die Sonde Folgendes umfasst:

   - ein einziges gemeinsames optisches Objektiv (10);
   - eine prismatische Monoblock-Separatorbaugruppe (40) und Spektralfilter (41,42);

   wobei das optische Objektiv, das Monoblock-Separatorprisma, die Spektralfilter, der erste und zweite fotoempfindliche Matrixsensor so ausgestattet sind, dass, wenn das optische Objektiv in einem vorbestimmten Abstand von der Eingriffszone angeordnet ist, das Bild im Fluoreszenzspektrum der gegebenen Zone durch das Objektiv auf der fotoempfindlichen Oberfläche des ersten Matrixsensors gebildet wird und das Bild im sichtbaren Spektrum der gegebenen Zone durch das Objektiv auf der fotoempfindlichen Oberfläche des zweiten Matrixsensors gebildet wird, wobei der Analysator dann zwischen dem Monoblock-Separatorprisma und dem zweiten fotoempfindlichen Matrixsensor angeordnet ist.

3. Optische Sonde nach Anspruch 2, wobei der erste Matrixsensor (51) mit einem ersten Filter (41) assoziiert ist, das nur im Fluoreszenzband durchlässt, und der zweite Matrixsensor (52) mit einem zweiten Filter (42) assoziiert ist, das sichtbare Wellenlängen mit Ausnahme von den im Fluoreszenzband enthaltenen durchlässt.

4. Optische Sonde für medizinische Anwendungen, so ausgestattet, dass sie in einer einzigen Hand gehalten werden kann, wobei die Sonde wenigstens Folgendes umfasst:

- eine erste Beleuchtungsquelle, genannt Anregungsbeleuchtungsquelle (20), ausgelegt zum Hervorrufen einer Fluoreszenzstrahlung von vorbestimmten Substanzen,
- eine zweite sichtbare Beleuchtungsquelle (21), wobei die erste und die zweite Quelle so ausgestattet sind, dass sie eine gemeinsame Zone (1), Eingriffszone genannt, beleuchten;
- ein optisches Objektiv (10);
- eine prismatische Monoblock-Separatorbaugruppe (40) und Spektralfilter (41, 42);
- einen fotoempfindlichen Matrixsensor (53);

wobei das optische Objektiv, das Monoblock-Separatorprisma, die Spektralfilter, der fotoempfindliche Matrixsensor so ausgestattet sind, dass, wenn das optische Objektiv in einem vorbestimmten Abstand von der Eingriffszone angeordnet ist, das Bild im Fluoreszenzspektrum der gegebenen Zone durch das Objektiv auf dem ersten Teil (54) der fotoempfindlichen Oberfläche des Matrixsensors gebildet wird, und das Bild im sichtbaren Spektrum der gegebenen Zone durch das Objektiv auf einem zweiten Teil (55) der fotoempfindlichen Oberfläche des Matrixsensors gebildet wird, wobei die zweite sichtbare Beleuchtungsquelle ein Polarisationsfilter umfasst, wobei ein Analysator dann oberhalb des zweiten Teils des fotoempfindlichen Matrixsensors angeordnet ist, wobei die Polarisationsrichtung des Analysators dann lotrecht zur Polarisationsrichtung des Polarisationsfilters ist, **dadurch gekennzeichnet, dass** die erste Beleuchtungsquelle, genannt Anregungsbeleuchtungsquelle (20), eine Laserquelle ist, deren Spektralemission dem Anregungsspektrum entspricht, und dadurch, dass die Sonde Mittel (80, 81) zum Messen der Neigung der optischen Sonde und Mittel (82) zum Beschneiden der Laserquelle (20) umfasst, wenn die Neigung der optischen Sonde einen vorbestimmten Wert überschreitet.

5. Optische Sonde nach Anspruch 4, **dadurch gekennzeichnet, dass** die prismatische Separatorbaugruppe einen Separatorwürfel (40) und ein Umlenkprisma (45) und eine Kompensationslamelle (46) umfasst, wobei der Separatorwürfel eine dichroitische Behandlung (44) umfasst, die die sichtbare Strahlung reflektiert und die im Fluoreszenzband befindliche Strahlung durchlässt oder umgekehrt.

6. Optische Sonde nach Anspruch 5, **dadurch gekennzeichnet, dass** die prismatische Separatorbaugruppe ein "Koster"-Prisma ist, gebildet aus zwei winkelmäßig identischen Prismen, wobei die den beiden Prismen gemeinsame Fläche eine dichroitische Behandlung (44) umfasst, die die sichtbare Strahlung reflektiert und die im Fluoreszenzband befindliche Strahlung durchlässt oder umgekehrt.

7. Optische Sonde nach einem der vorherigen Ansprüche, **dadurch gekennzeichnet, dass** die zweite Beleuchtungsquelle (30) wenigstens eine elektrolumineszente sogenannte "weiße" Diode ist, die ein Filter umfasst, das das Fluoreszenzspektrum beschneidet.

8. Optische Sonde nach Anspruch 7, **dadurch gekennzeichnet, dass** die zweite Beleuchtungsquelle mehrere filtrierte weiße Dioden umfasst, die regelmäßig um ein optisches Objektiv herum angeordnet sind.

9. Optische Sonde nach einem der vorherigen Ansprüche, **dadurch gekennzeichnet, dass** die Sonde eine Abbildungsvorrichtung (70) umfasst, so ausgestattet, dass sie entweder das Bild im sichtbaren Spektrum der Eingriffszone oder das Bild im Fluoreszenzspektrum der Eingriffszone oder eine Überlagerung dieser beiden Bilder anzeigt, wobei die Bilder von dem oder den fotoempfindlichen Matrixsensor(en) ausgegeben werden.

**Claims**

1. An optical probe for medical applications designed to be able to be held in one hand, said probe comprising at least:

- a first lighting source (20), called excitation lighting source, adapted to cause fluorescence radiation of predetermined substances;
- a second visible lighting source (21), the first and the second source being designed to illuminate a common zone (1), called intervention zone;
- a first photosensitive matrix sensor (51);
- a second photosensitive matrix sensor (52);

the first and second photosensitive matrix sensors being designed so that, when the probe is disposed at a predetermined distance from the intervention zone, the image in the fluorescence spectrum of said zone forms on the photosensitive surface of the first matrix sensor and the image in the visible spectrum of said zone forms on the photosensitive surface of the second matrix sensor, the second visible lighting source comprises a polarising filter, with an analyser then being disposed upstream of the second photosensitive matrix sensor, the polarisation direction of the analyser then being perpendicular to the polarisation direction of the polarising filter,
**characterised in that** the first lighting source (20), called excitation lighting source, is a laser source, the spectral emission of which corresponds to the excitation spectrum, and **in that** the probe comprises means (80, 81) for measuring the incline of the optical probe and means (82) for cutting off the laser source (20) when the incline of the optical probe exceeds a predetermined value.

2. The optical probe according to claim 1, said probe comprising:

   - a single shared optical lens (10);
   - a one-piece prismatic splitter assembly (40) and spectral filters (41,42);

   the optical lens, the one-piece splitter prism, the spectral filters, the first and second photosensitive matrix sensors being designed so that, when the optical lens is disposed at a predetermined distance from the intervention zone, the image in the fluorescence spectrum of said zone given by the lens forms on the photosensitive surface of the first matrix sensor and the image in the visible spectrum of said zone given by the lens forms on the photosensitive surface of the second matrix sensor, with the analyser then being disposed between the one-piece splitter prism and the second photosensitive matrix sensor.

3. The optical probe according to claim 2, such that the first matrix sensor (51) is associated with a first filter (41), transmitting only in the fluorescence band, and that the second matrix sensor (52) is associated with a second filter (42), transmitting visible wavelengths except for those included in the fluorescence band.

4. An optical probe for medical applications designed to be able to be held in one hand, said probe comprising at least:

   - a first lighting source (20), called excitation lighting source, adapted to cause fluorescence radiation of predetermined substances;
   - a second visible lighting source (21), the first and the second source being designed to illuminate a common zone (1) called intervention zone;
   - an optical lens (10);
   - a one-piece prismatic splitter assembly (40) and spectral filters (41, 42);
   - a photosensitive matrix sensor (53);

   the optical lens, the one-piece splitter prism, the spectral filters, the photosensitive matrix sensor being designed so that, when the optical lens is disposed at a predetermined distance from the intervention zone, the image in the fluorescence spectrum of said zone given by the lens forms on the first part (54) of the photosensitive surface of the matrix sensor and the image in the visible spectrum of said zone given by the lens forms on a second part (55) of the photosensitive surface of the matrix sensor, the second visible lighting source comprises a polarising filter, with an analyser then being disposed upstream of the second part of the photosensitive matrix sensor, the polarisation direction of the analyser then being perpendicular to the polarisation direction of the polarising filter,
**characterised in that** the first lighting source (20), called excitation lighting source, is a laser source, the spectral emission of which corresponds to the excitation spectrum, and **in that** the probe comprises means (80, 81) for measuring the incline of the optical probe and means (82) for cutting off the laser source (20) when the incline of the optical probe exceeds a predetermined value.

5. The optical probe according to claim 4, **characterised in that** the prismatic splitter assembly comprises a splitter cube (40) and a deflecting prism (45) and a compensation plate (46), the splitter cube comprising a dichroic treatment (44) reflecting the visible radiation and transmitting the radiation located in the fluorescence band, or vice versa.

6. The optical probe according to claim 5, **characterised in that** the prismatic splitter assembly is a "Koster" prism formed by two identical right-angle prisms, with the face common to the two prisms comprising a dichroic treatment (44) reflecting the visible radiation and transmitting the radiation located in the fluorescence band, or vice versa.

7. The optical probe according to any one of the preceding claims, **characterised in that** the second lighting source (30) is at least one light-emitting diode, called "white" diode, comprising a filter cutting off the fluorescence spectrum.

8. The optical probe according to claim 7, **characterised in that** the second lighting source comprises a plurality of filtered white diodes evenly disposed around an optical lens.

9. The optical probe according to any one of the preceding claims, **characterised in that** the probe comprises an imager (70) designed to display either the image in the visible spectrum of the intervention zone or the image in the fluorescence spectrum of the intervention zone or a superposition of these two images, said images originating from the one or more photosensitive matrix sensor(s).

# FIG. 1

FIG. 4

FIG. 5

## FIG. 6

Transmission normalisée

$R_L$

BG39

$E_L$

RG9

Longueur d'onde en nm

## FIG. 7

## RÉFÉRENCES CITÉES DANS LA DESCRIPTION

### Documents brevets cités dans la description

- US 20050182321 A
- WO 02061405 A
- US 2007232874 A
- US 4288158 A